(19) Europäisches Patentamt
European Patent Office
Office européen des brevets

(11) Veröffentlichungsnummer: **0 280 128**
**A2**

# (12) EUROPÄISCHE PATENTANMELDUNG

(21) Anmeldenummer: **88102069.7**

(51) Int. Cl.⁴: **C07H 19/073**

(22) Anmeldetag: **12.02.88**

(30) Priorität: **24.02.87 DE 3705794**

(43) Veröffentlichungstag der Anmeldung: **31.08.88 Patentblatt 88/35**

(84) Benannte Vertragsstaaten: **DE FR GB IT**

(71) Anmelder: **BAYER AG**
**Konzernverwaltung RP Patentabteilung**
**D-5090 Leverkusen 1 Bayerwerk(DE)**

(72) Erfinder: **Hayauchi, Yutaka, Dr.**
**Gustav-Freytag-Strasse 4**
**D-5090 Leverkusen(DE)**
Erfinder: **Lockhoff, Oswald, Dr.**
**Morgengraben 14**
**D-5000 Köln 80(DE)**

(54) **Verfahren zur Herstellung von 1-(3-Azido-2,3-didesoxy-beta-D-erythro-pentofuranosyl) pyrimidinen sowie neue Zwischenprodukte.**

(57) Die Erfindung betrifft ein neues Verfahren zur Herstellung von 1-(3-Azido-2,3-didesoxy-β-D-erythro-pentofuranosyl)pyrimidinen der allgemeinen Formel I

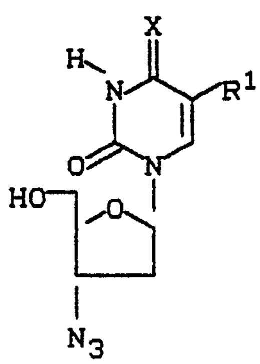

(I)

worin bedeutet:
X Sauerstoff, NH, $N-CO-CH_3$ oder $N-CO-C_6H_5$
$R^1$ Wasserstoff, Methyl, Ethyl oder Halogen
sowie neue Zwischenprodukte.

EP 0 280 128 A2

# Verfahren zur Herstellung von 1-(3-Azido-2,3-didesoxy-β-D-erythro-pentofuranosyl)pyrimidinen sowie neue Zwischenprodukte

Die Erfindung betrifft ein neues Verfahren zur Herstellung von 1-(3-Azido-2,3-didesoxy-β-D-erythro-pentofuranosyl)pyrimidinen der allgemeinen Formel I

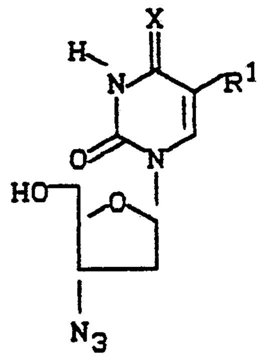

(I)

worin bedeutet:
X Sauerstoff, NH, $N\text{-}CO\text{-}CH_3$ oder $N\text{-}CO\text{-}C_6H_5$
$R^1$ Wasserstoff, Methyl, Ethyl oder Halogen
sowie neue Zwischenprodukte;
1-(3-Azido-2,3-didesoxy-β-D-erythro-pentofuranosyl)pyrimidine sind be4kannte antivirale Wirkstoffe. Insbesondere 3'-Azido-3'-desoxy-thymidin ist eine gegen menschliche Retroviren aktive Verbindung.

Einige Verfahren zur Herstellung von 3'-Azido-2',3'-didesoxy-pyrimidinnucleosiden sind bereits beschrieben worden. Diese Verfahren gehen von gut zugänglichen 2'-Desoxy-β-D-ribofuranosyl-pyrimidinen aus und beinhalten eine doppelte Konfigurationsumkehr an dem 3'-C-Atom. Man kann diese literaturbekannten Syntheseverfahren in zwei Gruppen einteilen.

Bei dem einen Verfahren wird zuerst 5'-O-geschütztes 3'-Chlor-bzw. 3'-O-Mesyl-D-threo-pentofuranosid isoliert und dies an der 3'-Position der zweiten nucleophilen Substitutionsreaktion mit Metallazid unterworfen. Nach Abspaltung der 5'-O-Schutzgruppe erhält man die 3'-Azido-2,3'-didesoxy-Verbindung. Beschrieben ist dieses Verfahren in:
J.P. Horwitz et al. J. Org. Chem., 29 (1964) 2076-8, T.S. Lin et al., J. Med. Chem., 21 (1978) 109-112, T.A. Krenitsky et al., J. Med. Chem., 26 (1983) 891-895.

Bei dem zweiten Verfahren nutzt man eine intramolekulare Substitutionsreaktion, der eine Stabilisierung durch Deprotonierung folgt. Das so erhaltene 2,3'-Anhydronucleosid läßt sich dann mit Metallazid umsetzen und weiter unter Öffnung der 2,3'-Anhydrobindung zu 1-(3-Azido-2,3-didesoxy-5-O-trityl-β-D-erythro-pentofuranosyl)thymin umsetzen.

Beschrieben sind diese Verfahren bei:
N.C. Miller et al., J. Org. Chem., 29, (1964) 1772-1776; A.M. Michelson et al., J. Chem. Soc., (1955) 816-823; J.J. Fox et al., J. Org. Chem., 28 (1963) 936-942; A. Joecks et al., J. Prakt. Chem. 325 (1983) 881-1048; G. Etzold, Tetrahedron, 27 (1971) 2463-72; J.A. Secrist III, Carbohydr. Res., 42 (1975) 379-381; T.S. Lin et al., J. Med. Chem., 26 (1983) 544-548; R.P. Glinski et al., J. Org. Chem., 38 (1973) 4299-4305 und J. Chem. Soc. Chem. Commun., (1970) 915-916.

Bei diesen bekannten Synthesewegen ist zur Cyclisierung starke Base wie Ammoniak, Natronlauge, Kalilauge, Natriummethanolat, 1,5-Diazabicyclo[5,4,0]-undec-5-en (DBU) oder Kaliumphthalimid notwendig. Durch diese Reaktionsbedingungen ist die Wahl der Schutzgruppe der 5'-Hydroxyfunktion auf basenstabile beschränkt. In Anwesenheit von starken Basen steigt auch die Gefahr von Nebenreaktionen, die zu einer Verminderung der Ausbeute führen.

Es wurde nun gefunden, daß sich die Synthese durch Verwendung neuer Zwischenprodukte und neuer Verfahrensschritte für die Bildungsreaktion der 2,3'-Anhydrobrücke wesentlich effektiver gestalten läßt als in der oben angeführten Literatur beschrieben. Durch das erfindungsgemäße Verfahren ist der Syntheseweg für die Verbindungen der Formel I kürzer und allgemeiner anwendbar geworden.

Die Erfindung betrifft ein Verfahren zur Herstellung von Verbindungen der allgemeinen Formel I

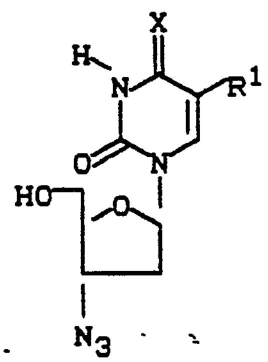

(I)

worin
X für Sauerstoff, NH, $N-CO-CH_3$ oder $N-CO-C_6H_5$
und
$R^1$ für Wasserstoff, Methyl, Ethyl oder Halogen steht,
welches dadurch gekennzeichnet ist, daß man eine Verbindung der Formel II

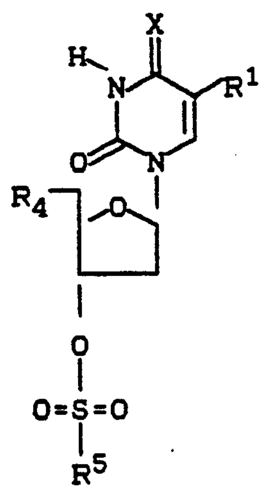

(II)

worin
$R^4$ für einen in eine Hydroxylgruppe überführbaren Substituenten oder für O-Y steht wobei Y eine O-Schutzgruppe darstellt
und
$R^5$ für Aryl, Alkyl oder Halogenalkyl steht,
in Anwesenheit von Fluoridsalzen oder schwachen Basen zum korrespondierenden 2,3'-Anhydroderivat cyclisiert
und
den 2,3'-Anhydroring durch Umsetzung mit einem Metallazid zum 3'-Azidoderivat spaltet, wobei die Überführung von $R^4$ in die Hydroxylgruppe bzw. die Abspaltung der O-Schutzgruppe Y vor oder nach der Ringöffnung stattfinden kann.

In dem erfindungsgemäßen Verfahren steht $R^4$ bevorzugt für Halogen oder $O-SO_2R^5$. $R^5$ bedeutet dabei bevorzugt Alkyl oder Halogenalkyl mit bis zu 6 C-Atomen. Ebenfalls bevorzugt für $R_5$ sind Phenylreste die durch $C_1$-$C_3$-Alkyl, Halogen oder Nitro substituiert sein können. Halogen steht für Fluor, Chlor oder Brom.

Die bevorzugte Bedeutung von Y ist Trityl, Mono-oder Dimethoxytrityl, Benzoyl, Pivaloyl, Benzyloxymethyl oder auch

$(R^3)_3Si$-

worin
$R^3$ verzweigtes oder unverzweigtes Alkyl mit bis zu 5 C-Atomen bedeutet oder für Aryl steht.

Besonders bevorzugt für $(R^3)_3Si$ ist tertiär-Butyldimethylsilyl.

Durchgeführt werden kann die Cyclisierung mit schwachen Basen oder bevorzugt mit Fluoridsalzen. Solche Fluoridsalze sind KF, NaF, $NH_4F$, $KHF_2$, $NaHF_2$, $NH_4HF_2$, CsF, Pyridiniumhydrogenfluorid oder

Tetrabutylammoniumfluorid.

Eine Verbindung die mit den erfindungsgemäßen Verfahren hergestellt werden kann ist das 3'-Azido-3'-desoxythymidin.

Die Erfindung betrifft auch neue Zwischenprodukte der Formel

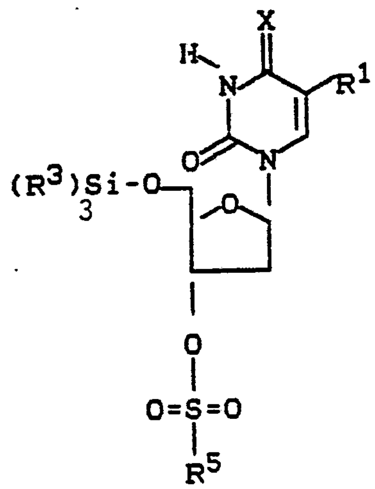

worin

X für Sauerstoff, NH, N-CO-$CH_3$ oder N-CO-$C_6H_5$.

$R^1$ für Wasserstoff, Methyl, Ethyl oder Halogen,

$R^3$ für verzweigtes oder unverzweigtes Alkyl mit bis zu 5 C-Atomen steht oder Aryl bedeutet und

$R^5$ für Aryl, Alkyl oder Halogenalkyl steht.

Bevorzugt betrifft die Erfindung solche Zwischenprodukte, in denen die Reste $R^3$ des Substituenten $(R^3)_3$Siunabhängig voneinander für einen Methyl-, tertiär Butyl-oder einen Phenylrest stehen, der durch $C_1$-$C_3$-Alkyl, Halogen oder Nitro substituiert sein kann. Halogen steht für Fluor, Chlor oder Brom.

Insbesondere betrifft die Erfindung Verbindungen der Formel

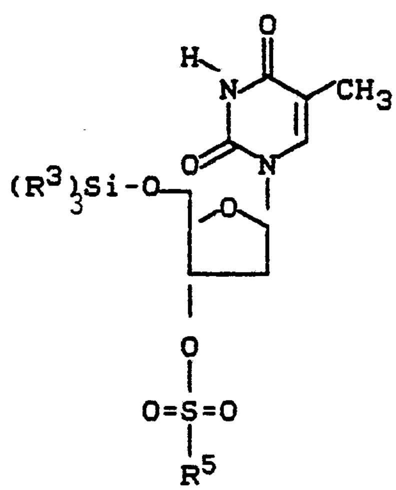

**worin**

$R^3$ für Alkyl mit bis zu 5 C-Atomen und

$R^5$ für Alkyl oder Halogenalkyl mit bis zu 5 C-Atomen steht.

Für $(R^3)_3$Si ist tert.-Butyldimethylsilyl und für $R^5$ sind Methyl oder Trifluormethyl als bevorzugt anzusehen.

Eine wesentliche Verbesserung, die das erfindungsgemäße Verfahren mit sich bringt, besteht in der Verwendung einer Silyletherschutzgruppe für die 5'-OH-Gruppe, der Sulfonylierung der verbleibenden 3'-OH-Gruppe mit Aryl-, Halogenalkyl-oder Alkylsulfonsäure und der Verwendung von Fluoridsalzen zur Abspaltung der Silyletherschutzgruppe bei gleichzeitiger Cyclisierung zur 2,3'-Anhydrozwischenstufe V.

Diese gesamte Reaktionsfolge ist im Reaktionsschema 1 dargestellt.

**Reaktionsschema 1**

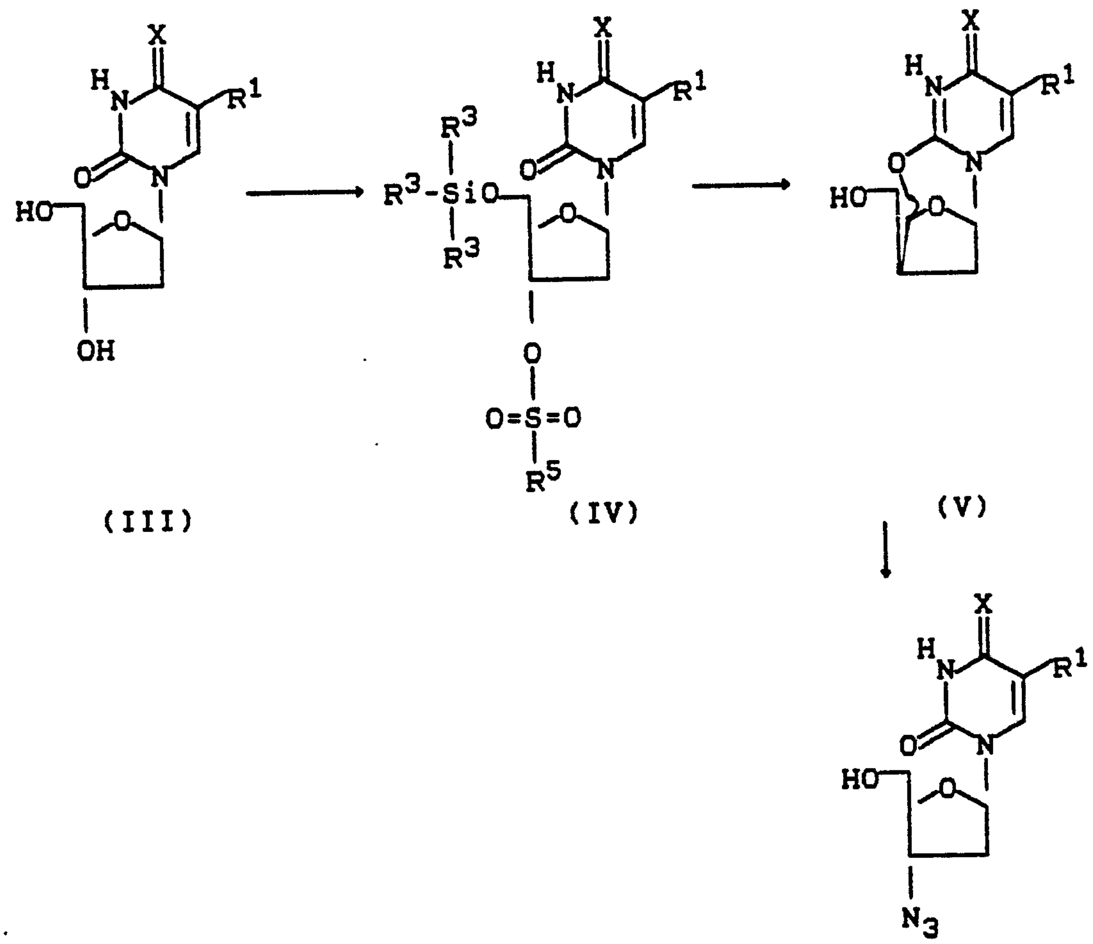

(III) (IV) (V) (I)

Hierin haben X und $R^1$ die obengenannten Bedeutungen, während $(R^3)_3Si$ für Trialkylsilyl oder Monoalkyldiarylsilyl steht. $R_5$ bedeutet Alkyl, Aryl oder Halogenalkyl.

Die Trialkyl-und Monoalkyldiarylsilyletherreste sind derart gewählt, daß das entsprechende Silylierungsreagenz die selektive Blockierung der primären Hydroxylfunktion auch bei Gegenwart freier sekundärer OH-Gruppen gewährleistet. Die Alkylreste des Trialkyl-und Monoalkyldiarylsilylrests sind gesättigte, gegebenenfalls ver zweigte aliphatische Alkylreste mit bis zu 5 C-Atomen und können im Fall von Trialkylsilyl auch voneinander verschieden sein. Beispiele für Trialkylsilylreste sind tert.-Butyldimethylsilyl und (1,2-Dimethylpropyl)dimethylsilyl. Monoalkyldiarylsilyl kann beispielsweise tert.-Butyldiphenylsilyl sein.

Ein wesentlicher Vorteil in dem erfindungsgemäßen Verfahren liegt, verglichen mit dem Stand der Technik, in einer durch den Silyletherrest und die Fluoridsalze ermöglichten milden und effektiven Katalyse der Cyclisierungsreaktion der 5'-O-Silyl-3'-O-methansulfonyl-Nucleoside zu 2,3'-Anhydro-Verbindungen (V). Die Ausbeutesteigerung und die Vereinfachung der Synthese solcher 2,3'-Anhydronucleoside und letzten Endes der 1-(3-Azido-2,3-didesoxy-β-D-erythro-pentofuranosyl)pyrimidine (I) werden auch dadurch erzielt, daß man mit zugesetztem Fluoridsalz in einem Reaktionsschritt sowohl die Silyletherschutzgruppe abspaltet, als auch die Cyclisierung erreicht.

Bekannt ist, daß Silylether durch Fluoridsalze zu Alkoholen gespalten werden. Ferner gibt es einen experimentellen Hinweis auf die Bildung des 2,3'-Anhydrothymidins aus dem 5'-OH-freien 3'-O-Methansulfonyl-thymidin (G. Etzold, oben zitiert). Diese Cyclisierungsreaktion tritt erst bei sehr hohen Temperaturen von mehr als 150° C auf.

Durch die Verwendung der 5'-O-Silyletherschutzgruppe wurde erreicht, daß die für die Darstellung der Zielverbindungen entscheidenden Cyclisierungsreaktionen allgemein anwendbar sind, mit guten Ausbeuten ablaufen und auch in großem Maßstab durchgeführt werden können.

Für die Cyclisierungsreaktion IV → V kann man übliche Fluoridsalze heranziehen, wie beispielsweise KF, NaF, $KHF_2$, $NaHF_2$, $NH_4F$, $NH_4F$, $NH_4HF_2$, CsF, Tetrabutylammoniumfluorid, Pyridiumhydrogenfluorid und andere. Bevorzugte Fluoridsalze sind Tetrabutylammoniumfluorid und KF.

Die Verwendung von Silylethergruppen in Verfahren zur Herstellung von 2,3'-Anhydronucleosiden der allgemeinen Struktur V ist neu. Sie bietet gegenüber den vorstehend erläuterten, bekannten Verfahrensweisen große Vorteile. Aufgrund des Einsatzes des Fluoridsalzes in Kombination mit der Verwendung der 5'-O-Silyl-3'-O-methansulfonyl-Nucleoside der allgemeinen Formel IV wird der Syntheseweg verkürzt und die Gesamtausbeute gesteigert.

Ein weiterer Vorteil ist auch, daß die 5'-O-Silylierung und die 3'-O-Methansulfonylierung in einem Einstufenverfahren durchgeführt werden können.

Aus der 2,3'-Anhydro-Verbindung V lassen sich dann beispielsweise nach dem literaturbekannten Verfahren 1-(3-Azido-2,3-didesoxy-β-D-erythro-pentofuranosyl)pyrimidine gut herstellen (M. Imazawa et al., J. Org. Chem., 43 (1978) 3044-3048).

Ein weiterer Vorteil der erfindungsgemäßen fluoridkatalysierten Cyclisierungsreaktion von IV zu V ist darin zu sehen, daß sie nicht nur auf 5'-O-silylierte Verbindungen beschränkt ist, sondern auch in Gegenwart anderer Substituenten an C-5' durchführbar ist. Dadurch ist es möglich, an C-5' unterschiedlich funktionalisierte 3'-O-Sulfonate der allgemeinen Formel VIIa generell durch milde Fluoridionen-Katalyse zu cyclisieren. Diese genannte Möglichkeit wird mit dem Reaktionsschema 2 erläutert.

## Reaktionsschema 2

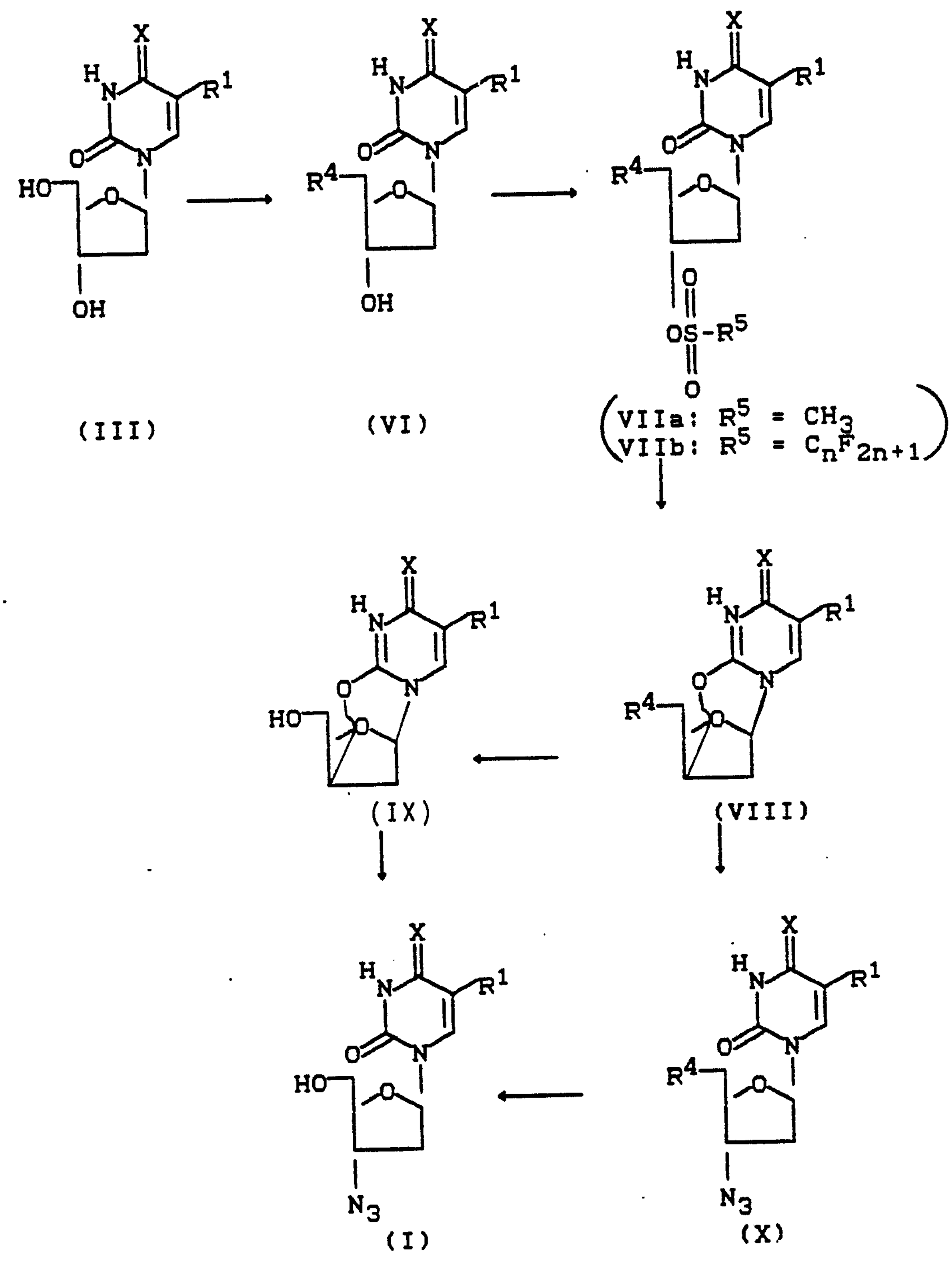

(III) (VI) (VIIa: $R^5 = CH_3$; VIIb: $R^5 = C_nF_{2n+1}$)

(IX) (VIII)

(I) (X)

Hierbei stehen $R^1$ und X für die vorstehend erläuterten Substituenten, während $R^4$ zum einen eine Gruppe -O-Y darstellt, in der Y eine in der Nucleosid-oder Kohlenhydratchemie übliche O-Schutzgruppe für primäre Hydroxylgruppen steht (siehe Protective Groups in Organic Chemistry, J.F.W. McOmie, Plenum Press, 1973).

Zum anderen schließt $R^4$ auch solche Substituenten ein, die in eine Hydroxylfunktion überführt werden können.

Die Schutzgruppen für 5'-OH sind beispielsweise Trityl, Mono-oder Dimethoxytrityl, Benzoyl, Pivaloyl, Benzyloxymethyl. Die in einer Folgereaktion zu Hydroxylgruppe überführbaren Substituenten $R^4$ sind beispielsweise Sulfonate $-OSO_2-R^5$ wie Tosylate oder Methansulfonate oder Halogenatome wie Chlor, Brom und Iod.

Wie das allgemeine Reaktionsschema 2 zeigt, kann die Öffnung des 2,3'-Anhydroringsystems durch Metallazide unter Bildung der 3'-Azido-Nucleoside vor oder nach der Abspaltung der 5'-O-Schutzgruppen bzw. der Überführung der Vorstufen $R^4$ in die OH-Funktion erfolgen. Die Entblockierungsmethoden für die oben angeführten Schutzgruppen sind bekannt (J.F.W. McOmie, oben zitiert).

Wiederherstellung der Hydroxylfunktion aus dem Substituent $R^4$ kann verschiedenartig durchgeführt werden. Beispielsweise kann die Vorstufe zuerst in ein 5'-O-Acetat oder 5'-O-Benzoat überführt und dies dann zu 5'-OH freiem Produkt gespalten werden.

Die im Reaktionsschema 2 dargestellte Reaktion macht von der Flexibilität der durch Fluoridsalz katalysierten Cyclisierungsreaktion Gebrauch und erweitert die funktionellen Gruppen am 5'-C der einzusetzenden 3'-O-Methansulfonyl-Nucleoside auch auf die vorstehend erläuterten Substituenten $R^4$. Aufgrund der effektiven und milden Katalyse des Fluoridsalzes ist es damit möglich geworden, daß funktionelle Gruppen $R^4$, die bei den herkömmlichen Verfahren nicht eingesetzt werden konnten oder keine befriedigende präparative Lösung darstellten, nach dem erfindungsgemäßen Verfahren Anwendung finden können. Das gilt vor allem für Acyloxygruppen wie Benzoyloxy oder Pivaloyloxy, für Halogene wie Chlor, Brom oder Iod, und für Sulfonate wie Tosylat oder Mesylat.

Das erfindungsgemäße Verfahren ermöglicht auch den Einsatz von Perfluoralkansulfonaten, wie es im Reaktionsschema 2 mit $R^5 = C_nF_{2n+1}$ für VIIb dargestellt ist. Hierbei kann n die Zahl von 1 bis 5, vorzugsweise 1 und 4, annehmen. $R^4$ im Schema 2 steht für die obengenannten Aralkyloxy-, Acyloxy-, substituierten Alkyloxy-, Sulfonyloxy-, Silyloxygruppen oder für Halogen.

Perfluoralkansulfonate zeichnen sich durch eine viel größere Tendenz aus, in nucleophilen Substituionsreaktionen als Austrittsgruppe zu fungieren, verglichen mit Methansulfonat-oder Tosylatgruppen. Diese stärkere Austrittsneigung der Perfluoralkansulfonate macht sich in diesem erfindungsgemäßen Verfahrem in einer leichteren Bildung der 2,3'-Anhydrostruktur VIII bemerkbar. Es ist also nicht mehr notwendig, starke Basen wie bei den herkömmlichen Verfahren zu verwenden. Vielmehr genügt es, wenn weit mildere Basen wie organische tertiäre Amine oder schwach basische anorganische Salze wie Hydrogencarbonate eingesetzt werden. Die oben erläuterten Fluoridsalze sind ebenso geeignet.

Geeignete Amine sind beispielsweise Trimethylamin, Triethylamin, Pyridin, 2,6-Dimethylpyridin oder 2,4,6-Trimethylpyridin. Beispiele für anorganische Hydrogencarbonate sind Natriumhydrogencarbonat, Kaliumhydrogencarbonat oder Ammoniumhydrogencarbonat.

Die Perfluoralkansulfonate ermöglichen es darüber hinaus, daß die Cyclisierungsreaktion bei tieferen Reaktionstemperaturen durchgeführt werden kann als bei den aus dem Stand der Technik bekannten Verfahren. Ferner gelingt die Cyclisierung auch mit solchen funktionellen Gruppen $R^4$, die bei den drastischeren Verfahrensbedingungen aus dem Stand der Technik ausgeschlossen sind.

Die Reaktivität der 3'-Perfluoralkansulfonate VIIb kann sich unter Umständen derart vergrößert haben, daß sich ausgehend von 5'-substituierten Nucleosiden VI die 3'-O-Perfluoralkansulfonyl-Verbindung VIIb gar nicht isolieren läßt, sondern daß durch die im Reaktionsmedium sofort eintretende Cyclisierung gleich das 2,3'-Anhydro-Nucleosid VIII bildet und als stabiles Reaktionsprodukt isoliert werden kann. Mit einer gut abgestimmten Reaktionsführung können die 5'-O-Blockierung, 3'-O-Perfluoralkansulfonierung und die Cyclisierung in einem vorteilhaften Eintopfverfahren durchlaufen werden.

Das bedeutet eine weitere Vereinfachung und Verbesserung des Verfahrens zur Herstellung der 1-(3-Azido-2,3-didesoxy-β-D-pentofuranosyl)pyrimidine (I). Die hier beschriebenen 3'-Perfluoralkansulfonate sind neu.

Im folgenden sollen die erfindungsgemäßen Herstellungsverfahren und mögliche Variationen näher erläutert werden.

Die hier eingesetzten Silyletherschutzgruppen lassen sich nach an sich schon bekannten Methoden einführen (J.F.W. McOmie, oben zitiert). Dazu setzt man Trialkyl-oder Monoalkyldiarylsilylchlorid mit 1-(2-Desoxy-β-D-erythro-pentofuranosyl)-pyrimidin der allgemeinen Formel III um.

Die Monosilylierung der freien Pyrimidin-Nucleoside III wird in Gegenwart einer organischen Base durchgeführt.

Geeignete organische Basen sind beispielsweise primäre, sekundäre oder tertiäre Amine wie Propylamin, Butylamin, Imidazol, Triazol, Triethylamin, Pyridin. Die Silylierungsreaktion kann sowohl lösungsmittelfrei als auch in einem wasserfreien inerten aprotischen Lösungsmittel durchgeführt werden, beispielsweise in N,N-Dimethylformamid (DMF), Dimethylsulfoxid (DMSO), Dioxan, Tetrahydrofuran (THF), Aceton, Methylenchlorid, Ethylenchlorid, Essigester oder Acetonitril.

Will man das 5'-O-Silyl-Nucleosid isolieren, sind selbst primäre und sekundäre Amine einsatzfähig. Ist dagegen die unmittelbare Weiterverarbeitung des Silyletherproduktes zu 5'-O-Silyl-3'-O-methansulfonyl-Verbindungen X beabsichtigt, dann ist der Einsatz tertiärer Amine als Base unbedingt erforderlich.

Die Temperatur, Reaktionsdauer und Einsatzmenge des Silylchlorids in bezug auf das Nucleosid

müssen derart aufeinander abgestimmt sein, daß nur der Monosilylether entsteht. Dazu setzt man Silylchlorid in 0,5 bis 5,0, vorzugsweise von 1,0 bis 3,0, Äquivalenten zum Nucleosid zu. Man steuert die Silylierung bei Temperaturen von -80°C bis 100°C, vorzugsweise von -50°C bis 50°C, dann endet die Reaktion innerhalb von 7 Tagen, vorzugsweise von 4 Tagen.

Das Silylierungsprodukt kann nach an sich üblichen Weisen aufgearbeitet und rein dargestellt werden.

Die Methansulfonylierung der 3'-OH-Funktion läßt sich nach an sich bekannten Verfahren durchführen (Synthetic Procedures in Nucleic Acid Chemistry, Herausgeber W.W. Zorbach und R.S. Tipson, John Wiley Sons, 1968).

Von Vorteil ist es, die nach der Silylierung folgende Methansulfonylierungsreaktion so anzuschließen, daß die Aufarbeitung des Silylierungsansatzes erspart bleibt. Dazu wird der Silylierungsansatz nach der Beendigung der Reaktion gekühlt, mit Methansulfonylchlorid versetzt und die Sulfonylierungsreaktion bei Temperaturen von 0°C bis zu 100°C, vorzugsweise bis zu 50°C, fortgesetzt. Die Sulfonylierungsreaktion wird dünnschichtchromatographisch verfolgt und kann 7 Tage, vorzugsweise 2 Tage dauern.

Man läßt dann auf das so hergestellte 5'-O-Silyl-3'-methansulfonat IV das oben beschriebene Fluoridsalz in einem geeigneten Lösungsmittel einwirken.

Man kann dabei wasserfreie Bedingungen nehmen, oder aber in wäßrigen Medien arbeiten.

Für die Fluoridsalz-katalysierten Cyclisierungsreaktion geeignete Lösungsmittel sind Kohlenwasserstoffe wie Toluol, Benzol oder Xylol, chlorierte Kohlenwasserstoffe wie Methylenchlorid, 1,2-Dichlorethan oder Chloroform, Ether wie Tetrahydrofuran (THF), Dioxan oder Diisopropylether, Ester wie Essigester, Ketone wie Aceton, Alkohole wie Methanol, Ethanol Isopropanol, Ethylenglykol, Diethylenglykol, Amide wie N,N-Dimethylformamid, Dimethylsulfoxid, Wasser oder Essigsäure, oder ein beliebiges Gemisch aus diesen Einzelkomponenten. Bevorzugt sind wasserfreies Tetrahydrofuran, und wäßriges Aceton.

Die Reaktionstemperatur hängt im wesentlichen von dem eingesetzten Fluoridsalz ab und kann von -20°C zu 150°C variieren, vorzugsweise von 0°C bis 100°C.

Unter den oben erläuterten Reaktionsbedingungen dauert diese Reaktion bis zu 2 Wochen, vorzugsweise 7 Tage.

Die einzusetzenden Fluoridsalzmengen können in bezug auf das 3'-Methansulfonat der Formel IV von einer äquimolareren bis zu 10fach äquimolaren Menge, vorzugsweise bis zur 5fach äquimolarer Menge, variieren.

Die Cyclisierungsansätze können nach üblichen Weisen aufgearbeitet und isoliert werden.

Von dem 2,3'-Anhydroprodukt der Formel V gelangt man nach literaturbekannten Verfahren zur Zielverbindung I (M. Imazawa, oben zitiert).

Reste $R^4$, die nucleophil substituierbare Gruppen wie Halogenen, Tosylat und Mesylat enthalten, müssen gesondert behandelt werden.

1-(2,5-Didesoxy-5-halogen-β-D-erythro-pentofuranosyl)pyrimidine mit Chlor, Brom und Iod für Halogen können beispielsweise nach Literaturverfahren synthetisiert werden (A.M. Michelson et al., J. Chem. Soc., (1955) 816, J.P.H. Verheyden et al., J. Org. Chem., 35 (1970) 2319).

1-(2-Desoxy-5-O-tosyl-β-D-erythro-pentofuranosyl)pyrimidine kann man nach an sich bekannten Methoden herstellen (J.P. Horwitz et al., J. Org. Chem., 27 (1962) 3045-3048).

1-(2-Desoxy-3,5-di-O-methansulfonyl-β-D-erythro-pentofuranosyl)pyrimidine kann beispielsweise durch an sich bekannte Methansulfonylierung von entsprechenden freien Pyrimidin-Nucleosiden erhalten werden (Synthetic Procedures in Nucleic Acid Chemistry, oben zitiert).

Diese 5'-substituierten 3'-O-Methansulfonyl-Nucleoside VIIa werden nach dem oben erläuterten Cyclisierungsverfahren zu 5'-substituierten 2,3'-Anhydroprodukten VIII überführt. Diese 5'-Substituenten der Nucleoside VIII lassen sich nucleophil zu 5'-O-Acetaten bzw. 5'-O-Benzoaten verwandeln, die dann zu Hydroxylfunktion der 2,3'-Anhydronucleoside IX verseift werden können. Der weitere Weg zum Endprodukt wurde bereits oben beschrieben.

Die Öffnung des 2,3'-Anhydroringes mit Metallazid kann genauso vor der Unwandlung der 5'-Substituenten in die Hydroxylgruppe vorgezogen werden, wie die Reaktionssequenz von VIII über X zu I zeigt.

Die Perfluoralkansulfonylierung der 3'-OH-Gruppe (Reaktion VI → VII im Schema 2) kann nach bekannten Methoden bewerkstelligt werden (G.W.J. Fleet et al., Tetrahedron Lett., 25 (1984) 4029-4032). Darüber hinaus kann 3'-Perfluoralkansulfonat der allgemeinen Formel VIIb auch wie folgt hergestellt werden. Wie das Reaktionsschema 2 zeigt, wird von 5'-O-geschützten bzw. 5'-substituierten Nucleosiden VI ausgegangen. Als Sulfonylierungsreagenz dient beispielsweise Perfluoralkansulfonsäureanhydrid wie Trifluormethansulfonsäureanhydrid oder Nonafluorbutansulfonsäureanhydrid. Hierbei wird als Base tertiäres Amin wie z.B. Triethylamin oder Pyridin zugesetzt und die Umsetzung in einem inerten aprotischen Lösungsmittel wie z.B. Dichlormethan, Dichlorethan, Tetrahydrofuran, Dioxan oder N,N-Dimethylformamid durchgeführt.

Besonders wichtig für erfolgreiche Herstellung dieser hochempfindlichen 3'-Perfluoralkansulfonate VIIb sind niedrig zu haltende Reaktionstemperatur und die Einsatzmengen sowohl des Perfluoralkansulfonsäureanhydrids als auch der Base.

Die Reaktionstemperatur wird zwischen -100°C und 25°C, vorzugsweise zwischen -70°C und 0°C, gehalten.

Die Einsatzmengen des Perfluoralkansulfonsäureanhydrids, bezogen auf die zu sulfonylierende Nucleosid-Vorstufe VI, liegen zwischen äquimolar und zweifach äquimolar, vorzugsweise zwischen äquimolar und anderthalbfach äquimolar.

Für die Einsatzmenge der Base gilt das Verhältnis von äquimolar bis dreifach äquimolar, vorzugsweise bis zweifach äquimolar.

Das so gebildete 5'-O-geschützte bzw. 5'-substituierte 3'-Perfluoralkansulfonat VIIb kann in an sich bekannter Weise rein dargestellt werden.

Die Cyclisierung dieser 3'-O-Perfluoralkansulfonate VIIb wird dann mit Fluoridsalz, in Gegenwart organischer tertiärer Amine oder anorganischer Salze wie Hydrogencarbonat durchgeführt.

Bei der basenkatalysierten Cyclisierung wird das 3'-O-Perfluoralkansulfonyl-Nucleosid VIIb in einem obengenannten Lösungsmittel umgesetzt.

Die Base wird bezogen auf 3'-Perfluoralkansulfonat von äquimolar bis zu zehnfach äquimolarer Menge, vorzugsweise bis zu zweifach äquimolar eingesetzt. Die Temperatur kann von -70°C bis 100°C, vorzugsweise von -30°C bis zu 50°C, variieren. Diese Cyclisierungsgreaktion endete dann in 7 Tagen, vorzugsweise 3 Tagen.

Man kann das 3'-Perfluoralkansulfonat VIIb isolieren und dann erneut der Cyclisierungsreaktion unterwerfen.

Vielfach erfolgt die Cyclisierung jedoch schon im Ansatz der Perfluoralkansulfonylierung, so daß man gleich als Reaktionsprodukt das 2,3'-Anhydronucleosid der allgemeinen Formel VIII gewinnt.

Diese Erfindung betrifft ebenso die in diesen Verfahren verwendeten Zwischenprodukte. Solche neuen Zwischenprodukte sind zum Beispiel:

5'-O-tert.-Butyldimethylsilyl-3'-O-methansulfonylthymidin,
5'-O-tert.-Butyldimethylsilyl-2'-desoxy-3'-O-methansulfonyl-uridin,
5'-O-tert.-Butyldimethylsilyl-2'-desoxy-3'-O-methansulfonyl-cytidin,
$N^4$-Acetyl-1-(5-O-tert-butyldimethylsilyl-2-desoxy-3-O-methansulfonyl-β-D-erythro-pentofuranosyl)cytosin,
$N^4$-Benzoyl-1-(5-O-tert.-butyldimethylsilyl-2-desoxy-3-O-methansulfonyl-β-D-erythro-pentofuranosyl)cytosin,
1-(5-O-tert.-Butyldimethylsilyl-2-desoxy-3-O-methansulfonyl-β-D-erythro-pentofuranosyl)-5-fluor-uracil,
5'-O-tert.-Butyldiphenylsilyl-3'-O-methansulfonyl-thymidin,
5'-O-tert.-Butyldiphenylsilyl-2'-desoxy-3'-O-methansulfonyl-uridin,
5'-O-tert.-Butyldiphenylsilyl-2'-desoxy-3'-O-methansulfonyl-cytidin,
$N^4$-Actyl-1-(5-O-tert.-butyldiphenylsilyl-2-desoxy-3-O-methansulfonyl-β-D-erythro-pentofuranosyl)cytosin,
$N^4$-Benzoyl-1-(5-O-tert.-butyldiphenylsilyl-2-desoxy-3-O-methansulfonyl-β-D-erythro-pentofuranosyl)cytosin,
1-(5-O-tert.-Butyldiphenylsilyl-2-desoxy-3-O-methansulfonyl-β-D-erythro-pentofuranosyl)-5-fluor-uracil,
5'-O-tert.-Butyldimethylsilyl-3'-O-trifluormethansulfonyl-thymidin,
5'-O-tert.-Butyldimethylsilyl-2'-desoxy-3'-O-trifluormethansulfonyl-uridin,
5'-O-tert.-Butyldimethylsilyl-2'-desoxy-3'-O-trifluormethansulfonyl-cytidin,
$N^4$-Acetyl-1-(5-O-tert.-butyldimethylsilyl-2-desoxy-3-O-trifluormethansulfonyl-β-D-erythro-pentofuranosyl)-cytosin,
$N^4$-Benzoyl-1-(5-O-tert.-butyldimethylsilyl-2-desoxy-3-O-trifluormethansulfonyl-β-D-erythro-pentofuranosyl)-cytosin,
1-(5-O-tert.-Butyldimethylsilyl-2-desoxy-3-O-trifluormethansulfonyl-β-D-erythro-pentofuranosyl)-5-fluor-uracil
5'-O-tert.-Butyldiphenylsilyl-3'-O-trifluormethansulfonylthymidin,
5'-O-tert.-Butyldiphenylsilyl-2'-desoxy-3'-O-trifluormethansulfonyl-uridin.
5'-O-tert.-Butyldiphenylsilyl-2'-desoxy-3'-O-trifluormethansulfonyl-cytidin,
$N^4$-Acetyl-1-(5-O-tert.-butyldiphenylsilyl-2-desoxy-3-O-trifluormethansulfonyl-β-D-erythro-pentofuranosyl)-cytosin,
$N^4$-Benzoyl-1-(5-O-tert.-butyldiphenylsilyl-2-desoxy-3-O-trifluormethansulfonyl-β-D-erythro-pentofuranosyl)-cytosin,
1-(5-O-tert.-Butyldiphenylsilyl-2-desoxy-3-O-trifluormethansulfonyl-β-D-erythro-pentofuranosyl)-5-fluor-uracil,
3'-O-Trifluormethansulfonyl-5'-O-trityl-thymidin 2'-Desoxy-3'-O-trifluormethansulfonyl-5'-O-trityl-uridin,
2'-Desoxy-3'-O-trifluormethansulfonyl-5'-O-trityl-cytidin,
$N^4$-Acetyl-1-(2-desoxy-3-O-trifluormethansulfonyl-5-O-trityl-β-D-erythro-pentofuranosyl)cytosin,
$N^4$-Benzoyl-1-(2-desoxy-3-O-trifluormethansulfonyl-5-O-trityl-β-D-erythro-pentofuranosyl)-cytosin,

1-(2-Desoxy-3-O-trifluormethansulfonyl-5-O-trityl-β-D-erythropentofuranosyl)-5-fluor-uridin,
5'-O-Benzoyl-3'-O-trifluormethansulfonyl-thymidin,
5'-O-Benzoyl-2'-desoxy-3'-O-trifluormethansulfonyl-uridin,
5'-O-benzoyl-2'-desoxy-3'-O-trifluormethansulfonyl-cytidin,
$N^4$-Acetyl-1-(5-O-benzoyl-2-desoxy-3-O-trifluormethansulfonyl-β-D-erythro-pentofuranosyl)cytosin,
$N^4$-Benzoyl-1-(5-O-benzoyl-2-desoxy-3-O-trifluormethansulfonyl-β-D-erythro-pentofuranosyl)cytosin,
1-(5-O-Benzoyl-2-desoxy-3-O-trifluormethansulfonyl-β-D-erythro-pentofuranosyl)-5-fluor-uridin,
5'-Chlor-5'-desoxy-3'-O-trifluormethansulfonyl-thymidin,
5'-Chlor-2',5'-didesoxy-3'-O-trifluormethansulfonyl-uridin,
5'-Chlor-2',5'-didesoxy-3'-O-trifluormethansulfonyl-cytidin,
$N^4$-Acetyl-1-(5-chlor-2,5-desoxy-3-O-trifluormethansulfonyl-β-D-erythro-pentofuranosyl)cytosin,
$N^4$-Benzoyl-1-(5-chlor-2,5-didesoxy-3-O-trifluormethansulfonyl-β-D-erythro-pentofuranosyl)cytosin,
1-(5-Chlor-2,5-didesoxy-3-O-trifluormethansulfonyl-β-D-erythropentofuranosyl)-5-fluor-uridin,
5'-Brom-5'-desoxy-3'-O-trifluormethansulfonyl-thymidin 5'-Brom-2',5'-didesoxy-3'-O-trifluormethansulfonyl-uridin,
5'-Brom-2',5'-didesoxy-3'-O-trifluormethansulfonyl-cytidin,
$N^4$-Acetyl-1-(5-brom-2,5-didesoxy-3-O-trifluormethansulfonyl-β-D-erythro-pentofuranosyl)cytosin,
$N^4$-Benzoyl-1-(5-brom-2,5-didesoxy-3-O-trifluormethansulfonyl-β-D-erythro-pentofuranosyl)cytosin,
1-(5-Brom-2,5-didesoxy-3-O-trifluormethansulfonyl-β-D-erythropento-furanosyl)-5-fluor-uridin,
5'-Desoxy-5'-iod-3'-O-trifluormethansulfonyl-thymidin, 2',5'-Didesoxy-5'-iod-3'-O-trifluormethansulfonyl-uridin,
2',5'-Didesoxy-5'-iod-3'-O-trifluormethansulfonyl-cytidin,
$N^4$-Acetyl-1-(2,5-didesoxy-5-iod-3-O-trifluormethansulfonyl-β-D-erythro-pentofuranosyl)cytosin,
$N^4$-Benzoyl-1-(2,5-didesoxy-5-iod-3-O-trifluormethansulfonyl-β-D-erythro-pentofuranosyl)cytosin,
1-(2,5-Didesoxy-5-iod-3-O-trifluormethansulfonyl-β-D-erythropentofuranosyl)-5-fluor-uridin,

## Beispiel 1

5'-O-tert.-Butyldimethylsilyl-thymidin

Thymidin (2,42 g, 10 mMol) wird in Pyridin (50 ml) gelöst und bei 0°C mit einer Lösung von tert.-Butyldimethylsilylchlorid (1,5 g, 10 mMol) in Methylenchlorid (20 ml) versetzt. Nach einem Tag wird eine weitere Portion von tert.-Butyldimethylsilylchlorid (1,5 g, 10 mMol) gegeben. Nach einem weiteren Tag wird der Ansatz auf Eis gegossen, 30 Minuten gerührt, mit Methylenchlorid extrahiert, die Methylenchlorid-Phase gewaschen mit wäßriger Kaliumhydrogensulfat-, Natriumhydrogencarbonat-Lösung und Wasser, getrocknet und eingeengt, Ausbeute 3,1 g (88 %).

## Beispiel 2

$N^4$-Benzoyl-5'-O-tert.-butyldimethylsilyl-2'-desoxy-cytidin

$N^4$-Benzoyl-2'-desoxy-cytidin (3,3 g, 10 mMol) und Imidazol (2,0 g, 30 mMol) werden in Dimethylsulfoxid (10 ml) und N,N-Dimethylformamid (50 ml) gelöst, auf -40°C gekühlt und tropfenweise mit einer Lösung von tertiärem Butyldimethylsilylchlorid (1,8 g, 12 mMol) in DMF (10 ml) versetzt. Man setzt die Reaktion bei dieser Temperatur fort und kontrolliert den Ablauf dünnschichtchromatographisch (Methylenchlorid:Methanol 10:1). Nach Ende der Monosilylierung gibt man Wasser (1 ml) zu, rührt nach, engt die Lösung am Hochvakuum ein, und extrahiert den zurückbleibenden Sirup mit Methylen chlorid und Wasser, Nach Trocknung und Eindampfen der Methylenchloridphase wird das Produkt säulenchromatographisch (Methylenchlorid:Methanol 15:1) gereinigt, Ausbeute 3,8 g (85 %).

## Beispiel 3

5'-O-tert.-Butyldimethylsilyl-3'-O-methansulfonyl-thymidin

Thymidin (24,2 g, 100 mMol) wird in abs. Pyridin (150 ml) gelöst, auf 0°C gekühlt und tropfenweise (30 Min.) mit einer Lösung von tert.-Butyldimethylsilyl-chlorid (18,0 g, 120 mMol) in abs. Methylenchlorid (20 ml) versetzt. Der Ansatz wird dann bei Raumtemperatur 18 Stunden gerührt und nochmal mit tert.-Butyldimethylsilylchlorid (1,5 g, 10 mMol) hinzugefügt. Nach weiteren sechs Stunden wird die Reaktionslösung auf 0°C gebracht und Methansulfonsäurechlorid (8,0 ml, 100 mMol), gelöst in abs. Methylenchlorid (20 ml), zugegeben. Danach läßt die Badtemperatur langsam auf Raumtemperatur kommen und nach 24 Stunden wird die Reaktionslösung in Eis gegossen, gut gerührt und dreimal mit Methylenchlorid extrahiert. Die vereinigten Methylenchloridphasen werden getrocknet, eingedampft, nochmal in Methylenchlorid aufgenommen, diese mit wäßriger Kaliumhydrogensulfat-, Natriumhydrogencarbonat-Lösung und Wasser gewaschen, getrocknet und eingedampft. Das Produkt kristallisiert aus Methylenchlorid und n-Hexan, Ausbeute 28,5 g (65 %),
Schmelzpunkt 140-142°C $[\alpha]_D^{20}$ = +6,4° (C=1,15, Aceton) $^1$H-NMR (360 MHz, $CDCl_3$): $\delta$ = 0,17 (S, 6H, $CH_3$-Si), 0,97(S, 9H, $(CH_3)_3$C-Si), 1,97 (d, $J_{6,CH_3}$ = 1,2 Hz, 3H,$CH_3$-5-C), 2,24 (ddd, $J_{1',2''}$= 9,2 Hz, $J_{2',2'}$ = 14,3 Hz, $J_{2'',3'}$ = 6,1 Hz, 1H, 2"-H), 2,65 (ddd, $J_{1',2'}$ = 5,4 Hz, $J_{2',3'}$ = 1,2 Hz, 1H, 2'-H), 3,16 (S,3H, $CH_3$-S), 3,96 (m, 2H, 5'-H, 5"-H), 4,41 (quart. $J_{3',4'}$ = 1,6 Hz, $J_{4',5'}$ = 1,6 Hz, $J_{4',5''}$ = 1,6 Hz, 1H, 4'-H), 5,33 (ddd, 1H, 3'-H), 6,44 (dd, 1H, 1'-H), 7,52 (d, 1H, 6-H), 9,22 (breit S, 1H, NH).
$C_{17}H_{30}N_2O_7SSi$ (434.6)
Ber. C 47,0 H 7,0 N 6,4
Gef. C 47,0 H 7,0 N 6,4

Beispiel 4

5'-O-tert.-Butyldimethylsilyl-2'-desoxy-3'-O-methansulfonyl-uridin

2-Desoxyuridin (2,28 g, 10 mMol) liefert nach der Reaktionsdurchführung analog zu dem Beispiel 3 das Produkt, Ausbeute 3,23 g (77 %), Schmelzpunkt 104-105°C, $[\alpha]_D^{20}$ = +3.9° (C = 1,245, Aceton). $^1$H-NMR (360 MHz, $CDCl_3$): $\delta$ = 0,14 (S, 3H, $CH_3$-Si), 0,15 (S, 3H, $CH_3$-Si), 0,95 (S, 9H, $(CH_3)_3$C-Si), 2,25 (ddd, $J_{2',2''}$= 14,3 Hz, $J_{1',2''}$ = 8,3 Hz, $J_{2'',3'}$ = 5,9 Hz, 1H, 2"-H), 2,69 (ddd, $J_{1',2'}$ = 5,6 Hz, $J_{2',3'}$ = 1,6 Hz, 1H, 2'-H), 3,14 (S, 3H, $CH_3$-S), 3,93 (m, 2H, 5'-H, 5'-H), 4,41 (ddd, $J_{3',4'}$ = 2,0 Hz, $J_{4',5'}$ = 2,0 Hz, $J_{4',5''}$ = 2,0 Hz, 1H, 4'-H), 531 (ddd, 1H, 3'-H), 5,76 (dd, $J_{5,6}$ = 8,1 Hz, 5-H), 6,41 (dd, 1H, 1'-H), 7,85 (d, 1-H, 6-H), 8,98 (breit S, 1H, NH).
$C_{16}H_{28}N_2O_7SSi$ (420,5)
Ber. C 45,7 H 6,7 N 6,7
Gef. C 45,7 H 6,7 N 6,7

Beispiel 5

Methode a)

2,3'-Anhydro-1-(2-desoxy-$\beta$-D-threo-pentofuranosyl)thymin

Herstellung mit wasserfreiem Tetrabutylammoniumfluorid

Die Verbindung des Beispiels 3 (4,3 g, 10 mMol) wird in abs. Tetrahydrofuran (THF) (200 ml) gelöst, mit einer wasserfreien 1 N-Lösung von Tetrabutylammoniumfluorid in THF (15 ml, 15 mMol) versetzt und anderthalb Stunden auf 80°C erhitzt. Der Ansatz wird dann abgekühlt, mit Methylenchlorid verdünnt und mit Wasser extrahiert. Nach Eindampfen der wäßrigen Lösung wird der Sirup mit Ethanol verrüht und das Produkt kristallisiert. Ausbeute 1,7 g (81 %), Schmelzpunkt 231 bis 233°C,
$[\alpha]_D^{20}$ = -12,7° (C = 0,84, Wasser).
$^1$H-NMR (360 MHz, $CD_3OD$): $\delta$ = 1,92 (d, $J_{6CH_3}$ = 1,2 Hz, 3H, $CH_3$-C=), 2,59 (ddd, $J_{1',2''}$ = 3,7 Hz, $J_{2',2''}$ = 13,1 Hz, $J_{2'',3'}$ = 2,7 Hz, 1H, 2"-H), 2,66 (dd, $J_{1',2'}$ = 0 Hz, $J_{2',3'}$ = 1,6 Hz, 1H, 2'-H), 3,69 (dd, $J_{4',5''}$ = 6,3 Hz, $J_{5',5''}$ = 11,7 Hz, 1H, 5"-H), 3,74 (dd, $J_{4',5'}$ = 6,3 Hz, 1H, 5'-H), 4,34 (ddd, $J_{3',4'}$ = 2,6 Hz, 1H,

4'-H), 5,34 (ddd, 1H, 3'-H), 5,83 (d, 1H, 1'-H), 7,53 (quart., 1H, 6-H).
$C_{10}H_{12}N_2O_4$ (224,2)
Ber. C 53,6 H 5,4 N 12,5
Gef. C 53,6 H 5,4 N 12,5

Beispiel 6

Methode b)

2,3'-Anhydro-1-(2-desoxy-β-D-theo-pentofuranosyl)thymin

Herstellung mit Kaliumfluorid

Die Verbindung des Beispiels 3 (430 mg, 1 mMol) wird in Aceton (3 ml) und Wasser (0,5 ml) gelöst, mit Kaliumfluorid (290 mg, 5 mMol) versetzt und fünf Tage unter Rückfluß gekocht. Nach Abkühlen wird den Ansatz eingedampft und das Produkt kristallisiert aus Ethanol, Ausbeute 190 mg (56 %).

Beispiel 7

2,3'-Anhydro-1-(2-desoxy-β-D-threo-pentofuranosyl)uracil

Die Verbindung des Beispiels 4 (1,75 g, 4,2 mMol) liefert nach der Reaktionsdurchführung analog zum Beispiel 5 das Produkt, Ausbeute 550 mg (62 %), Schmelzpunkt 193-195°C $[\alpha]_D^{20}$ = -3,8° (C = 0,895, Wasser)
$^1$H-NMR (360 MHz, $CDCl_3$): δ = 2,54 (dd, $J_{2',2''}$ = 13,0 Hz, $J_{1',2''}$ = 0 Hz, $J_{2'',3'}$ = 1,5 Hz, 1H, 2''-H), 2,61 (ddd, $J_{1',2'}$ = 3,0 Hz, $J_{2',3'}$ = 2,4 Hz, 1H, 2'-H), 3,71 (dd, $J_{5',5''}$ = 11,5 Hz, $J_{4',5''}$ = 7,1 Hz, 1H, 5''-H), 3,81 (dd, $J_{4',5'}$ = 6,1 Hz, 1H, 5'-H), 4,53 (ddd, $J_{3',4'}$ = 2,0 Hz, 1H, 4'-H), 5,41 (ddd, 1H, 3'-H), 5,96 (d, $J_{5,6}$ = 7,3 Hz,1 Hz, 5-H), 5,86 (d, 1H, 1'-H), 7,48 (d, 1H, 6-H).
$C_9H_{10}N_2O_4$ (210,2)
Ber. C 51,4 H 4,8 N 13,3
Gef. C 51,4 H 4,8 N 13,3

Beispiel 8

2,3'Anhydro-1-(2-desoxy-5-O-methansulfonyl-β-D-threo-pentofuranosyl)-thymin

3',5'-Di-O-methansulfonyl-thymidin (10,4 g, 31,3 mMol) wird in abs. Tetrahydrofuran (140 ml) gelöst, mit einer wasserfreien 1 N-Lösung von Tetrabutylammoniumfluorid in Tetrahydrofuran (31,3 ml, 31,3 mMol) versetzt und 24 Stunden auf 80°C erhitzt. Dann wird der Ansatz mit Methylenchlorid verdünnt, viermal mit Wasser extrahiert, die wäßrigen Phasen eingeengt und der zurückbleibende Sirup mit Isopropanol verrührt, Ausbeute 6,34 g (67 %), Schmelzpunkt 187 bis 189°C, $[\alpha]_D^{20}$ = +38,1° (C = 0,945, Wasser)
$^1$H-NMR (360 MHz, $D_2O$): δ = 1,88 (S, 3H, $CH_3$-C=), 2,67 (m, 2H, 2'-H, 2''-H), 3,11 (S, 3H, $CH_3S$), 4,54 (m, 1H, 4'-H), 5,49 (S, 1H, 3'-H), 5,96 (S, 1H, 1'-H), 7,57 (S, 1H, 6-H).
$C_{11}H_{14}N_2O_6S$ (302,3)
Ber. C 43,7 H 4,7 N 9,3
Gef. C 43,6 H 4,7 N 9,3

Beispiel 9

2,3'-Anhydro-1-(5-O-tert.-butyldimethylsilyl-2-desoxy-β-D-threopentofuranosyl)thymin

5'-O-tert.-Butyldimethylsilyl-thymidin (3,6 g, 10 mMol) wird in abs. Methylenchlorid (20 ml) gelöst, auf -78°C gekühlt und mit Trifluormethansulfonsäuranhydrid (1,85 ml, 11 mMol) versetzt. Dazu tropft man eine Lösung von Pyridin (8,05 ml, 100 mMol) in abs. Methylenchlorid (10 ml) über eine Stunde, hält die Lösung

noch fünf Stunden bei dieser Temperatur, läßt sie dann langsam auf Raumtemperatur steigen und setzt die Reaktion noch weitere zwei Tage fort. Danach versetzt man mit Wasser (2 ml), engt den Ansatz ein, und reinigt ihn säulenchromatographisch (Methylenchlorid:Methanol 15:1), Ausbeute 2,54 g (75 %), Sirup, $[\alpha]^{20}_{D}$ = -39,2° (C = 0,50, Aceton)
$^1H$-NMR (360 MHz, $CDCl_3$): $\delta$ = 0,05 (S, 3H, $CH_3Si$), 0,07 (S, 3H, $CH_3Si$), 0,87 (S, 9H, $(CH_3)_3$ Si), 1,93 (d, $J_6,CH_3$ = 1,2 Hz, 3H, $CH_3$-C=) 2,44 (ddd, $J_{1'},_{2''}$ = 4,0 Hz, $J_{2'},_{2''}$ = 12,8 Hz, $J_{2''},_{3'}$ = 2,8 Hz, 1H, 2''-H), 2,64 (dd, $J_{1'},_{2'}$ = 0 Hz, 1H, 2'-H), 3,74 (dd, $J_{4'},_{5''}$ = 7,4 Hz, $J_{5'},_{5''}$ = 10,6 Hz, 1H, 5''-H), 3,81 (dd, $J_{4'},_{5'}$= 6,0 Hz, 1H, 5'-H), 4,28 (ddd, $J_{3'},_{4'}$ = 2,4 Hz, 1H, 4'-H), 5,18 (ddd, 1H, 3'-H), 5,46 (d, 1H, 1'-H), 6,93 (quart., 3H, 6-H).
$C_{16}H_{26}N_2O_4Si$ (338,5)
Ber. C 56,8 H 7,7 N 8,3
Gef. C 56,7 H 7,7 N 8,3

Beispiel 10

3'-Azido-3'-desoxy-thymidin

2,3'-Anhydro-1-(2-desoxy-$\beta$-D-threo-pentofuranosyl)thymin (20,0 g, 0,125 Mol) wird in N,N-Dimethylformamid (600 ml) gelöst, mit Natriumazid (52 g, 0,80 Mol) versetzt und fünf Stunden auf 140°C erhitzt. Danach wird der Ansatz filtriert, eingeengt, in Wasser aufgenommen, die wäßrige Phase mehrmals mit Essigester extrahiert, die Essigesterphasen vereinigt, getrocknet, und eingeengt. Der zurückbleibende Sirup wird in Essigester und Methanol mit Aktivkohle behandelt und aus Wasser kristallisiert, Ausbeute 27,0 g, (81 %), Schmelzpunkt 118 bis 120°C.

## Ansprüche

1. Verfahren zur Herstellung von Verbindungen der allgemeinen Formel I

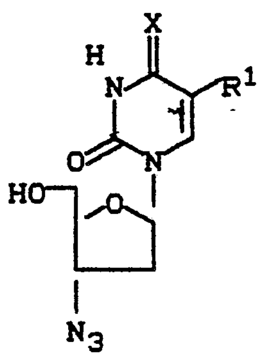

(I)

worin
X für Sauerstoff, NH, N-CO-$CH_3$ oder N-CO-$C_6H_5$
und
$R^1$ für Wasserstoff, Methyl, Ethyl oder Halogen steht,
dadurch gekennzeichnet, daß man eine Verbindung der Formel II

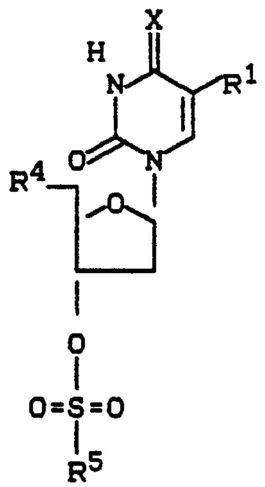

(II)

worin

$R^4$ für einen in eine Hydroxylgruppe überführbaren Substituenten oder für O-Y steht wobei Y eine O-Schutzgruppe darstellt

und

$R^5$ für Aryl, Alkyl oder Halogenalkyl steht,

in Anwesenheit von Fluoridsalzen oder schwachen Basen zum korrespondierenden 2,3'-Anhydroderivat cyclisiert

und

den 2,3'-Anhydroring durch Umsetzung mit einem Metallazid zum 3'-Azidoderivat spaltet, wobei die Überführung von $R^4$ in die Hydroxylgruppe bzw. die Abspaltung der O-Schutzgruppe Y vor oder nach der Ringöffnung stattfinden kann.

2. Verfahren gemäß Anspruch 1 worin $R^4$ für Halogen oder $-O-SO_2-R^5$ steht.

3. Verfahren gemäß Anspruch 1, worin Y für Trityl, Mono-oder Dimethoxytrityl, Benzoyl, Pivaloyl, Benzyloxymethyl oder für

$(R^3)_3Si$-steht,

worin

$R^3$ verzweigtes oder unverzweigtes Alkyl mit bis zu 5 C-Atomen bedeutet oder für Aryl steht.

4. Verfahren gemäß Anspruch 3, worin $(R^3)_3Si$ für tert.-Butyldimethylsilyl steht.

5. Verfahren gemäß den Ansprüchen 1 bis 4, worin $R^5$ für Alkyl oder Halogenalkyl mit bis zu 6 C-Atomen steht.

6. Verfahren gemäß den Ansprüchen 1 bis 4, worin $R^5$ für einen Phenylrest steht, der durch $C_1$-$C_3$-Alkyl, Halogen oder Nitro substituiert sein kann.

7. Verfahren gemäß den Ansprüchen 1 bis 6, worin Halogen Fluor, Chlor oder Brom bedeutet.

8. Verfahren gemäß den Ansprüchen 1 bis 7, worin die Cyclisierung durch KF, NaF, $NH_4F$, $KHF_2$, $NaHF_2$, $NH_4HF_2$, CsF, Pyridiniumhydrogenfluorid oder Tetrabutylammoniumfluorid katalysiert wird.

9. 3'-Azido-3'-desoxy-thymidin, hergestellt mit dem Verfahren gemäß den Ansprüchen 1 bis 8.

10. Verbindung der Formel

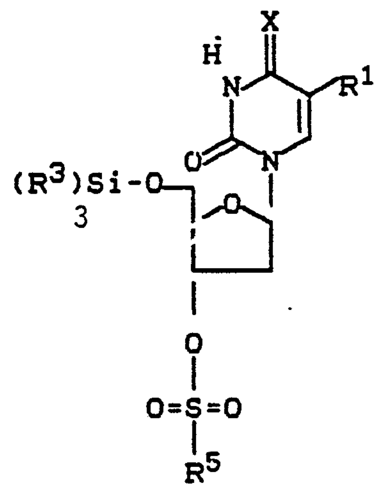

worin
X für Sauerstoff, NH, N-CO-$CH_3$ oder N-CO-$C_6H_5$,
$R^1$ für Wasserstoff, Methyl, Ethyl oder Halogen,
$R^3$ für verzweigtes oder unverzweigtes Alkyl mit bis zu 5 C-Atomen steht oder Aryl bedeutet und
$R^5$ für Aryl, Alkyl oder Halogenalkyl steht.

11. Verbindung gemäß Anspruch 10, worin $R^3$ für einen Phenylrest steht der durch $C_1$-$C_3$-Alkyl, Halogen oder Nitro substituiert sein kann.

12. Verbindung gemäß den Ansprüchen 10 und 11, worin Halogen für Fluor, Chlor und Brom steht.

13. Verbindung der Formel

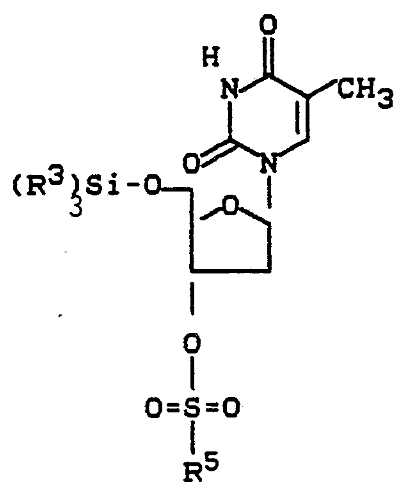

worin
$R^3$ für Alkyl mit bis zu 5 C-Atomen und
$R^5$ für Alkyl oder Halogenalkyl mit bis zu 5 C-Atomen steht.

14. Verbindung gemäß Anspruch 13, worin $(R^3)_3Si$ für tert.-Butyldimethylsilyl und für $R_5$ für Methyl oder Trifluormethyl steht.